(19) Europäisches Patentamt / European Patent Office / Office européen des brevets

(11) **EP 1 430 831 B1**

(12) **EUROPEAN PATENT SPECIFICATION**

(45) Date of publication and mention
of the grant of the patent:
**03.12.2014 Bulletin 2014/49**

(51) Int Cl.:
*A61B 5/00* *(2006.01)*      *A61L 2/00* *(2006.01)*
*C12Q 1/00* *(2006.01)*

(21) Application number: **03258046.6**

(22) Date of filing: **19.12.2003**

(54) **Method for manufacturing a sterilized and calibrated biosensor-based medical device**

Verfahren zur Herstellung einer sterilisierten und calibrierten medizinischen, biosensoren-basierten Vorrichtung

Procédé pour la fabrication d'un dispositif médical stérilisé et calibré comprennant des biosondes

(84) Designated Contracting States:
**AT BE BG CH CY CZ DE DK EE ES FI FR GB GR HU IE IT LI LU MC NL PT RO SE SI SK TR**

(30) Priority: **20.12.2002 US 324933**

(43) Date of publication of application:
**23.06.2004 Bulletin 2004/26**

(73) Proprietor: **LifeScan, Inc.**
**Milpitas, CA 95835 (US)**

(72) Inventor: **Teodorczyk, Maria**
**San Jose, CA 95135 (US)**

(74) Representative: **Tunstall, Christopher Stephen et al**
**Carpmaels & Ransford LLP**
**One Southampton Row**
**London WC1B 5HA (GB)**

(56) References cited:
**EP-A- 0 757 246**      **EP-A- 1 293 574**
**US-A1- 2002 023 852**      **US-A1- 2002 168 290**

• **Collison M.E. et al, "Analytical Characterization of Electrochemical Biosensor Test Strips for Measurement of Glucose in Low-Volume Interstitial Fluid Samples", Clinical Chemistry, 45:9, 1665-1673, 1999**

EP 1 430 831 B1

**Description**

BACKGROUND OF THE INVENTION

1. Field of the Invention

This invention relates, in general, to methods for the manufacturing of medical devices and, in particular, to methods for manufacturing sterilized and calibrated medical devices.

2. Description of the Related Art

Radiation-based sterilization of specific types of medical devices is common and widespread today due to both favorable economics and reliability. Depending on the type of medical device to be sterilized, radiation-based sterilization can be accomplished using either electromagnetic or particle radiation. Ionizing radiation in the electromagnetic spectrum (e.g., gamma [γ], x-ray and electron radiation) can produce bactericidal effects by transferring photon energy into characteristic ionizations in or near a biological target (e.g., detrimental microorganisms). In addition to the pairs of positive and negative ions that are created by such characteristic ionizations, free radicals and activated molecules can also be produced in medical devices undergoing radiation-based sterilization.

Gamma radiation has been commonly used to sterilize non-bioactive medical devices, including common hospital supplies such as plastic hypodermic syringes and sutures. Gamma radiation can successfully destroy detrimental microorganisms without increasing the temperature of the medical device undergoing radiation-based sterilization. Therefore, radiation-based sterilization that utilizes gamma radiation is often referred to as "cold sterilization." A minimum standard dose of 25 kGy of radiation has been routinely used in medical device sterilization. This dose can provide a safety factor equivalent to $10^{-6}$ inactivation of the most resistant microorganisms.

Exposure to radiation-induced energy can alter chemicals, including water, by prompting their ionization, decomposition and the production of free radicals. In the presence of oxygen, such free radicals can form hydrogen peroxide and/or hydroperoxyl radicals that act as oxidizing or reducing agents. These agents can subsequently degrade and otherwise alter a variety of chemicals and biochemicals (e.g., enzymes).

Gamma sterilization could be considered appropriate for complete destruction of microbial flora in biosensor-based medical devices (e.g., disposable glucose sensors which combine lancing, sample transfer and glucose concentration measuring components in a single integral medical device). However, sterilization of biosensor-based medical devices containing analyte specific reagents (i.e., biosensor reagent compositions such as analyte specific enzymes and associated mediators) has not heretofore been successful due to the fact that radiation can induce a detrimental effect on biosensor reagent compositions. This detrimental effect can alter the biosensor's chemistry resulting in an inaccurate response during use.

Ideally, biosensor-based medical devices should be sterilized as an assembled and packaged product. Otherwise, a less economic approach of sterilizing individual components of the biosensor-based medical device followed by assembly and packaging of the device under clean and sterile conditions would be necessary.

Analytical characterization of electrochemical biosensor test strips for measurement of glucose in low-volume interstitial fluid samples by M. Collison et al., Clinical Chemistry 45:9 1665-1673 (1999) describes that conventional single-use, electrochemical glucose test strip and ISF collection technologies can be readily integrated to provide real-time ISF sampling and glucose measurements for diabetic monitoring applications.

Still needed in the field, therefore, is a simple and inexpensive method for manufacturing a biosensor-based medical device that yields a biosensor-based medical device that is both sterile and accurately calibrated. In addition, the method should enable the sterilization of an assembled and packaged biosensor-based medical device.

SUMMARY OF THE INVENTION

Embodiments according to the present invention include methods for manufacturing a biosensor-based medical device that yields a biosensor-based medical device that is both sterile and accurately calibrated. In addition, the method enables the sterilization of an assembled and packaged biosensor-based medical device.

A method for manufacturing a sterilized and calibrated biosensor-based medical device according to one exemplary embodiment of the present invention is recited in claim 1.

Processes according to exemplary embodiments of the present invention provide for the manufacturing of a sterile biosensor-based medical device in an inexpensive manner by avoiding costs associated with assembling previously sterilized biosensor-based medical device components in a clean/sterile environment. Furthermore, highly accurate biosensor-based medical devices result from performing the sterilization step prior to the calibration step.

BRIEF DESCRIPTION OF DRAWINGS

A better understanding of the features and advantages of the present invention will be obtained by reference to the following detailed description that sets forth illustrative embodiments, in which the principles of the invention are utilized, and the accompanying drawings of which:

FIG. 1 is a perspective view of a biosensor-based medical device (i.e., an electrochemical biosensor-based medical device) that can be utilized in certain embodiments of present invention;

FIG. 2 is a perspective view of another biosensor-based medical device (i.e., a colorimetric/photometric biosensor-based medical device) that can be utilized in certain embodiments of the present invention;

FIG. 3 is a flow chart illustrating a sequence of steps in a process according to one exemplary embodiment of the present invention; and

FIG. 4 is a flow chart illustrating a sequence of steps in a process according to another exemplary embodiment of the present invention.

DETAILED DESCRIPTION OF THE INVENTION

Processes according to exemplary embodiments of the present invention can be employed to manufacture a variety of sterilized and accurately calibrated biosensor-based medical devices, including, but not limited to, the integrated biosensor and lancet medical devices described in U.S. Patent Application US 20020168290 A1.

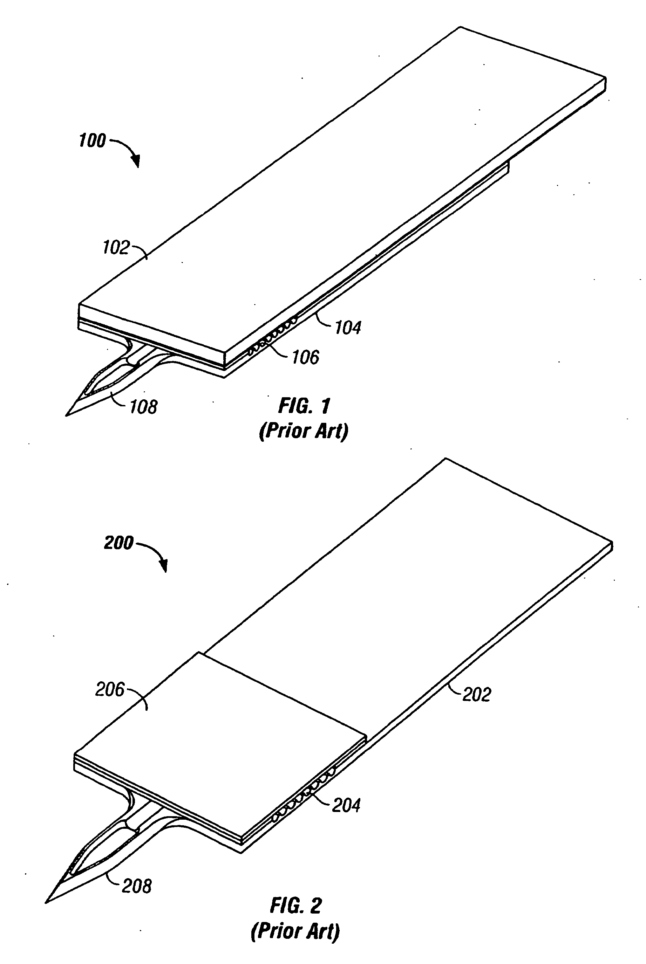

FIGs. 1 and 2 illustrate an electrochemical biosensor-based medical device and a colorimetric/photometric biosensor-based medical device, respectively, that can, for example, be manufactured by processes according to exemplary embodiments of the present invention.

Referring to FIG. 1, electrochemical biosensor-based medical device 100 includes a top electrode 102 and bottom electrode 104. The top electrode 102 and the bottom electrode 104 are held together by an adhesive layer (not shown). The adhesive layer is adapted to provide a reaction zone 106. Electrochemical biosensor-based medical device 100 also includes an integrated micro-needle 108 (also referred to as a lancet or an integrated lancet).

Furthermore, electrochemical biosensor-based medical device 100 includes a biosensor reagent composition (such as a redox reagent composition, not shown) present within reaction zone 106. The biosensor reagent composition is selected to interact with targeted component(s) (e.g., glucose) in a fluid sample (e.g., a whole blood sample) during an assay of the fluid sample. In electrochemical biosensor-based medical device 100, the biosensor reagent composition is disposed on top electrode 102 and resides within reaction zone 106.

In the configuration of FIG. 1, bottom electrode 104 is adapted to serve as a counter/reference electrode, while top electrode 102 is adapted to serve as a working electrode of an electrochemical cell. However, in other electrochemical biosensor-based medical device embodiments, and depending on a voltage sequence applied to the electrochemical cell, the role of the top and bottom electrodes can be reversed such that bottom electrode 104 serves as a working electrode, while top electrode 102 serves as a counter/reference electrode.

Suitable biosensor reagent compositions for electrochemical biosensor-based medical device 100 include, for example, an enzyme and a redox active component (e.g., a mediator). Further details related to electrochemical biosensor-based medical device 100 are discussed in EP-A-1 360 931.

FIG. 2 illustrates a colorimetric/photometric biosensor-based medical device 200 that includes a support substrate 202 made of an inert material, a matrix 204 for receiving a sample, a biosensor reagent composition (not illustrated) within matrix 204 that typically includes one or more members of an analyte oxidation signal producing system, and a top layer 206 (for example, a transparent top layer) which covers at least matrix 204. In other embodiments of a colorimetric/photometric biosensor-based medical device, top layer 206 can be, for example, a membrane containing a biosensor reagent composition impregnated therein, in which circumstance matrix 204 and the top layer 206 are mutually inclusive. Colorimetric/photometric biosensor-based medical device 200 also includes an integrated micro-needle 208 (also referred to as a lancet or an integrated lancet).

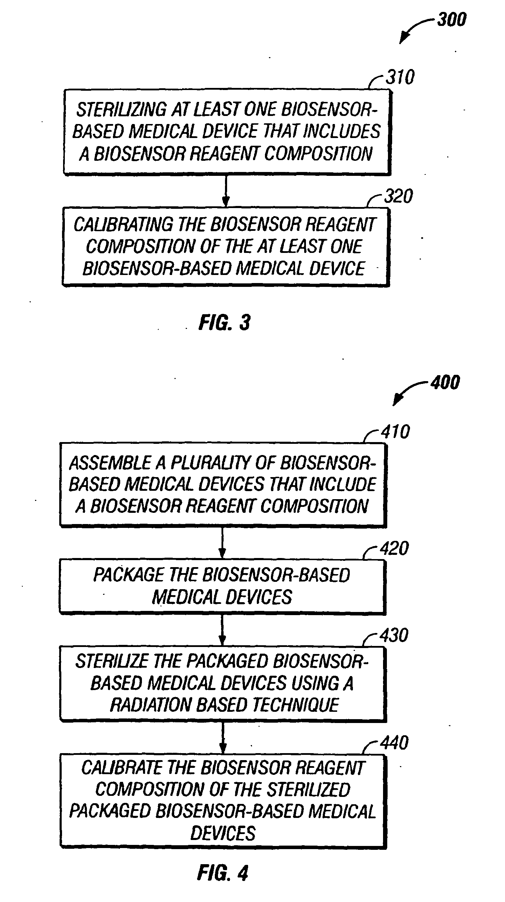

FIG. 3 is a flow chart illustrating a sequence of steps in a process 300 according to the present invention for manufacturing a sterilized and calibrated biosensor-based medical device. Process 300 includes the step of sterilizing at least one biosensor-based medical device (e.g., the medical devices of FIGs. 1 and 2 that include integrated lancets and biosensors, i.e., electrochemical and colorimetric/photometric sensors) to create at least one sterilized biosensor-based medical device, as set forth in step 310. The biosensor-based medical device(s) sterilized in step 310 includes a biosensor reagent composition.

Once apprised of the present disclosure, one skilled in the art will recognize that the present invention can be employed during the manufacturing of a variety of biosensor-based medical devices including, but not limited to, integrated biosensor and lancet devices described in EP-A-1 360 931.

Gamma sterilization can be considered appropriate for the complete destruction of harmful microbial flora in integrated biosensor and lancet devices that combine lancing, sample transfer and glucose concentration measuring (biosensor) components in a single integral disposable device. In such devices, a micro-needle is adapted to penetrate a subcutaneous skin layer, to access a blood sample and to transfer the blood sample to, for example, an electrochemical cell area of the device for glucose concentration determination. Therefore, the micro-needle must be provided in a sterile condition.

Process 300 is particularly beneficial for manufacturing a biosensor-based medical device that includes a biosensor reagent composition (e.g., a reagent composition that includes an analyte specific enzyme and associated mediator) whose analytical performance is altered upon exposure to radiation. For example, the analytical performance of a biosensor reagent composition that includes PQQ-based glucose dehydrogenase (a glucose specific enzyme) and ferricyanide (a mediator) has been determined as being altered by exposure to gamma radiation.

Sterilization step 310 can utilize any suitable sterilization technique. However, as will be described in detail below, processes according to exemplary embodiments of the present invention prove particularly useful when a radiation-based technique (e.g., a gamma radiation-based technique) is employed. Gamma radiation from a $Co^{60}$ source and a dose of 10 to 30 kGy can, for example, be used in sterilization step 310.

Next, the biosensor reagent composition of the at least one sterilized biosensor-based medical device is calibrated, as set forth in step 320. In order to avoid analytical inaccuracies resulting from changes in the analytical performance of a biosensor reagent composition due to sterilization step 310 (e.g., changes in calibration coefficients due to exposure of the biosensor reagent composition to gamma radiation), calibration step 320 is performed after sterilization step 310.

By performing calibration step 320 after sterilization step 310, effects of the sterilization step on the analytical performance of the biosensor-based medical device are compensated. For example, gamma radiation employed in a radiation-based sterilization technique can have an altering effect on the analytical performance of biosensor reagent compositions that include an analyte specific enzyme and a mediator. However, by conducting a calibration step subsequent to sterilization, such effects are compensated for during the calibration, thus providing an accurately calibrated biosensor-based medical device. This type of compensation can be particularly useful for integrated biosensor-based medical devices where a biosensor (e.g., an electrochemical cell biosensor or a colorimetric/photometric biosensor) and lancet are fabricated as a single integrated biosensor-based medical device.

FIG. 4 is a flow chart illustrating a sequence of steps in a process 400 according to the present invention for manufacturing a sterilized and calibrated biosensor-based medical device. Process 400 includes the step of assembling a plurality of biosensor-based medical devices, as set forth in step 410. The biosensor-based medical devices assembled in step 410 can be any suitable biosensor-based medical devices known to those skilled in the art. Process 400 is, however, particularly beneficial for manufacturing biosensor-based medical devices with a biosensor reagent composition and an integrated lancet, including those illustrated in FIGs. 1 and. 2.

Assembly of the biosensor-based medical device can be accomplished using any suitable assembly technique known to those skilled in the art including, but not limited to, those described in EP-A-1 360 931.

Next, at step 420, the biosensor-based medical devices assembled in step 410 are packaged to create packaged, biosensor-based medical devices. Such packaging encompasses, for example, cartridge form packages or individually wrapped devices in a card format package.

The packaged biosensor-based medical devices are then sterilized using a radiation-based sterilization technique, to create a plurality of sterilized, packaged biosensor-based medical devices, as set forth in step 430. In the circumstance that the biosensor-based medical devices include an integrated lancet, the sterilization step 430 is adapted to create a sterile lancet.

Next, the biosensor reagent composition of the sterilized, packaged biosensor-based medical devices are calibrated, as set forth in step 440. Only a fraction of a biosensor reagent composition batch used to assemble the plurality of biosensor-based medical devices need be used for the calibration step. For example, a sample (e.g., a statistically selected sample) of the sterilized, packaged biosensor-based medical devices can be calibrated versus a reference method. In this manner, calibration information (e.g., calibration coefficients) can be economically obtained for the remaining devices that were not part of the sample. In addition, calibration step 440 does not necessarily require clean/sterile room conditions, thereby not unduly increasing manufacturing cost.

Process 400 creates a sterile biosensor-based medical device in an inexpensive manner by avoiding costs associated with assembling previously sterilized components of a biosensor-based medical device (e.g., a previously sterilized lancet and an electrochemical test cell or photometric test strip) in a clean/sterile room. Furthermore, by performing sterilization prior to calibration, a highly accurate biosensor-based medical device is rendered.

In both process 300 and process 400, a sterilization step precedes a calibration step. This particular sequential order of steps (i.e., a sterilization step prior to a calibration step) enables the manufacturing of a sterilized and calibrated biosensor-based medical device of high accuracy and range, as demonstrated by Examples 1 and 2 below.

EXAMPLE 1: Effect of Gamma Radiation on the Enzyme Activity of a Biosensor Reagent Composition

Palladium (Pd) sputtered polyester panels (available from CP Films, Canoga Park, CA) were coated with a glucose sensitive biosensor reagent composition containing pyrroloquinoline quinone-glucose dehydrogenase (PQQ-GDH), pyrroloquinoline quinone (PQQ), potassium ferricyanide, a buffer and other components as set forth in Table 1 below. This biosensor reagent composition is described further in EP Application No. 03255680.5.

Table 1:

| Biosensor Reagent Composition | | |
| --- | --- | --- |
| **Component** | **Weight (g) in 100 mL** | **% solids** |
| Buffer (citraconate 66.7 mM): Citraconic acid | 0.0273 | 0.0869 |
| Buffer (buffer pH 6.8): Dipotassium Citraconate | 1.334 | 4.247 |
| Wetting agent (0.066 %): Pluronic P103 | 0.067 | 0.213 |
| Detergent (0.0332 %): Pluronic F87 | 0.033 | 0.105 |
| Enzyme stabilizer (1.7 mM): $CaCl_2$ | 0.019 | 0.0605 |
| Stabilizer (75 mM): Sucrose | 2.5673 | 8.174 |
| Enzyme Cofactor (484 $\mu$M): PQQ | 0.016 | 0.051 |
| Enzyme (240 $\mu$M): PQQ-GDH | 2.647 | 8.428 |
| Mediator (750 mM): Potassium Ferricyanide | 24.697 | 78.635 |
| Total solids: | 31.407 | 100.000 |

Dried Pd panels (size 6" by 1.5") coated with the biosensor reagent composition of Table 1 were packaged in KAPAK (Minneapolis, MN) pouches (1 panel per pouch) with silica gel desiccant and sealed under argon (Ar). The pouched samples were shipped to a sterilization facility together with a pouched control sample (i.e., a panel packaged in KPAK but that was not to be irradiated). A Gammacell 220 (serial no. 254) was used to irradiate (i.e., sterilize using a radiation-based technique) the samples. For this purpose, $Co^{60}$ was used as a source of gamma radiation. Sterilization was performed at Johnson & Johnson Sterilization Sciences & Technology (New Brunswick, NJ).

Following sterilization with 10, 20 and 30 kGy doses of gamma radiation (without opening the pouches), the samples were returned and the PQQ-GDH activity assayed using the DCIP/PES (DCIP = 2,6-Dichlorophenolindophenol Sodium salt, PES = phenazine ethosulfate) spectrophotometric method disclosed in EP Application No. 03255680.5.

The 10, 20 and 30 kGy doses where chosen based on a belief that a 25 kGy dose of gamma radiation is commonly used in medical device industry. It was assumed, therefore, that a 25 kGy dose would be sufficient to produce a suitably sterile biosensor-based medical device, however no analysis of microorganism concentration following the radiation-based sterilization was conducted. Once apprised of the present disclosure, suitable radiation doses for use in processes according to the present invention can be readily determined by one skilled in the art without undue experimentation.

A Pd panel sample freshly coated with the biosensor reagent composition of Table 1 was prepared. Table 2 below shows the effect of the dose of gamma radiation on the activity of PQQ-GDH enzyme for each of the samples described above.

Table 2: Effect of gamma radiation on activity of the PQQ-GDH enzyme coated Palladium Panel samples.

| Sample Type | Radiation Exposure Time (min.) | Recovered Enzyme Activity (U/mL) | Coefficient of Variation % (n = 6) | % Change from radiation free sample |
| --- | --- | --- | --- | --- |
| Fresh sample | N/A | 23.6 | 3.4 | N/A |
| Control sample (not irradiated but shipped to and from the sterilization facility) | N/A | 24.1 | 1.5 | N/A |
| 10 kGy | 48.9 | 21.0 | 1.7 | - 12.9 |

(continued)

| Sample Type | Radiation Exposure Time (min.) | Recovered Enzyme Activity (U/mL) | Coefficient of Variation % (n = 6) | % Change from radiation free sample |
|---|---|---|---|---|
| 20 kGy | 97.8 | 21.9 | 1.1 | - 9.1 |
| 30 kGy | 146.7 | 20.6 | 2.0 | - 14.5 |

The data of Table 2 indicate a degradation of the biosensor reagent composition's enzyme activity following gamma radiation in comparison to samples that were not subjected to gamma radiation. If desired, such an activity degradation (loss of activity) can be inexpensively compensated by depositing a reagent composition with an enzyme activity that is higher in proportion to the expected loss due to gamma radiation sterilization. For example, for a 30 kGy gamma radiation dose, a reagent composition with a 15% higher enzyme activity could be employed to compensate for the expected 14.5% enzyme activity loss.

EXAMPLE 2: Effect of Calibrating Biosensor-based Medical Devices Before and After a Sterilization Step

Fully assembled and ready-for-use glucose biosensor-based medical devices including the reagent composition of Table 1 and gold and palladium electrodes located in an opposed configuration were obtained. Prior to gamma radiation sterilization, these devices were calibrated by testing with blood samples containing plasma equivalent glucose concentrations of 30, 270 and 620 mg/dL, as measured by a reference-instrument method using a standard YSI instrument (commercially available from Yellow Springs, Ohio). The calibration tests included blood samples with low, normal and high hematocrit levels (i.e., 20%, 42% and 70 % hematocrit levels, respectively).

The biosensor reagent composition calibration step relies on collecting the response of multiple devices to blood samples of known plasma glucose concentration over a desired dynamic range (e.g., 20 - 600 mg/dL) and correlating the response to a reference method by minimizing differences between the two glucose readings. Ideally, the bias between the blood glucose concentration obtained from the biosensor-based medical device and from the glucose reference method for all blood samples should be zero. However, depending on glucose concentration and blood hematocrit, the bias can be non-zero (for example, up to $\pm$ 15 %). Typically, the following equation is obtained once a batch of biosensor-based medical devices have been calibrated:

$$\text{Glucose}_{YSI} = (\text{Glucose}_{sensor})^{a} + b$$

where:

"Glucose$_{YSI}$" is the glucose concentration as determined by the YSI reference instrument;
"Glucose$_{sensor}$" = glucose concentration as determined by a biosensor-based medical device;
"a" = a coefficient which brings sensor response in-line with glucose concentration determined by the reference method; and
"b" = an offset (intercept) coefficient (observed, for example, when a glucose free blood sample is tested); the "b" coefficient an be either a positive or a negative number.

The calibration step described above rendered the following values of coefficients: a = 0.6921 and b = 0.5854, when performed prior to a sterilization step. Calibrated biosensor-based medical devices were packaged into KAPAK pouches containing silica gel desiccant, sealed and divided into four groups: (i) stored in the package at a controlled temperature and humidity environment (i.e., 20-25 °C and <10% relative humidity), (ii) shipment control, (iii) sterilized with 20 kGy dose, and (iv) sterilized with 25 kGy dose.

The last three groups of biosensor-based medical devices (i.e., groups [ii]-[iv]) were shipped to the same sterilization facility as in Example 1. Following radiation exposure, a blood glucose test was performed according to the same protocol as in the calibration step, using the a and b coefficients derived from the calibration step performed before sensor sterilization. Table 3 shows the averaged response of biosensor-based medical devices tested with 20, 42 and 70 % hematocrit blood at three glucose concentrations (YSI values) and the bias of averaged response in mg/dL for the low glucose concentration or in % for the other two glucose concentrations.

Table 3: Response of glucose sensors sterilized at 20 and 25 kGy gamma radiation using calibration coefficients obtained by performing a calibration prior to sterilization (a = 0.6921, b = 0.5854); n = 18.

| Case | YSI Glucose (mg/dL) | Avg. Sensor Glucose (mg/dL) | Bias to YSI (mg/dL, or %) |
|---|---|---|---|
| 20 kGy | 32.7 | 44.5 | 11.8 |
|  | 266.3 | 270.5 | 1.57 |
|  | 606.0 | 565.8 | - 6.64 |
| 25 kGy | 32.7 | 45.1 | 12.4 |
|  | 266.3 | 268.3 | 0.73 |
|  | 606.0 | 564.2 | - 6.90 |
| Shipment Control | 32.7 | 28.1 | - 4.56 |
|  | 266.3 | 259.5 | - 2.56 |
|  | 606.0 | 571.0 | - 5.77 |
| Stored in Controlled Environment | 32.7 | 27.5 | - 5.18 |
|  | 266.3 | 264.6 | - 0.65 |
|  | 606.0 | 571.1 | - 5.76 |

The data of Table 3 indicate that, as an effect of sterilization using gamma radiation, a significant positive response bias at low glucose concentration is observed, rendering the biosensor-based medical devices relatively inaccurate at the glucose level where determination of hypoglycemia is critical to the patient treatment. On average, the YSI bias of devices irradiated at 20 and 25 kGy was about 12 mg/dL at the low (30 mg/dL) glucose concentration, whereas the bias of the shipping control and the sample stored in a controlled environment was only about - 5 mg/dL.

Although no additional analysis has been performed, except for a measurement of the device background response, a conjecture based on the enzyme activity change reported in Example 1 is that the primary source of the increase in response bias is the formation of potassium ferrocyanide from the oxidized form of the mediator.

Next, the calibration procedure was performed following the gamma radiation process to demonstrate that a biosensor-based medical device of improved accuracy is obtained. Such a process sequence accounts for analytical performance changes resulting from interaction of the gamma rays with the biosensor reagent composition, thus delivering a biosensor reagent composition with an accurate response throughout the whole dynamic range of the system. Table 4 below contains the response of biosensor-based medical devices that were calibrated following the gamma radiation step.

Table 4: Response of glucose sensors irradiated at 20 and 25 kGy using calibration coefficients derived following radiation sterilization.

| a = 0.7885, b = 1.088 for the 20 kGy dosage; a = 0.7974, b = 1.1242 for the 25 kGy dosage; n = 18. | | | |
|---|---|---|---|
| Case | YSI Glucose (mg/dL) | Avg. Sensor Glucose (mg/dL) | Bias to YSI (mg/dL, or %) |
| 20 kGy | 32.7 | 32.9 | 0.18 |
|  | 266.3 | 275.8 | 3.56 |
|  | 606.0 | 601.0 | - 0.82 |
| 25 kGy | 32.7 | 32.9 | 0.27 |
|  | 266.3 | 274.4 | 3.02 |
|  | 606.0 | 603.4 | - 0.43 |

The results of Table 4 demonstrated a significant improvement in bias to YSI in comparison to Table 3, especially at the lowest glucose concentration. Thus, if the reagent calibration step is performed following radiation sterilization, the response bias to the reference method is minimized because the calibration parameters determined during calibration reflect (compensate) any changes in biosensor reagent chemistry.

It is speculated, without being bound, that gamma rays cause formation of ferrocyanide $[Fe(CN)_6]^{-4}$ from the biosensor reagent composition mediator $[Fe(CN)_6]^{-3}$. When a blood sample is tested on the biosensor-based medical device, an increase in reduced mediator concentration is interpreted by the device as additional glucose. In other words, gamma radiation of the biosensor-based medical device is speculated to affect enzyme activity and/or integrity of the mediator, generating quantities of product that are mistakenly detected as an analyte by the device, thus compromising the device's accuracy. However, if during manufacturing biosensor-based medical devices are irradiated first and calibrated following the sterilization step, the effect of radiation is compensated for rendering a highly accurate biosensor-based medical device.

Since a major response shift is observed in the intercept portion of the calibration following gamma radiation, the biosensor reagent composition can be calibrated in the last manufacturing step, thus avoiding costly clean room assembly procedures. In summary, when a sterilization step is performed prior to a calibration step, the bias seen in a process with the sequence reversed is not present.

It should be understood that various alternatives to the embodiments of the invention described herein may be employed in practicing the invention. It is intended that the following claims define the scope of the invention and that methods within the scope of these claims be covered thereby.

**Claims**

1. A method for manufacturing sterilized and calibrated disposable integrated biosensor-based medical devices, the method comprising:

   assembling a plurality of disposable integrated biosensor-based medical devices each including a biosensor and a lancet integrated theretogether, the biosensor including a biosensor reagent composition;
   packaging the disposable integrated biosensor-based medical devices, thereby creating packaged disposable integrated biosensor-based medical devices;
   thereafter sterilizing the assembled packaged disposable integrated biosensor-based medical devices, thereby creating a plurality of sterilized, packaged disposable integrated biosensor-based medical devices;
   thereafter, compensating for the effects of the sterilization step on the analytical performance of the packaged disposable integrated biosensor-based medical devices by calibrating the biosensor reagent composition of the sterilized, packaged disposable integrated biosensor-based medical devices, the calibrating step utilizing a fraction of the sterilized, packaged disposable integrated biosensor-based medical devices to obtain calibration information.

2. The method of claim 1, wherein the integrated biosensor and lancet medical devices are electrochemical biosensor-based medical devices.

3. The method of claim 1, wherein the integrated biosensor and lancet medical device are photometric biosensor-based medical devices.

4. The method of any one of claims 1 to 3, wherein the sterilizing step utilizes a radiation-based sterilization technique.

5. The method of claim 4 wherein the sterilizing step utilizes a gamma radiation based sterilization technique.

6. The method of claim 4, wherein the sterilizing step utilizes a gamma radiation dose in the range of 10 kGy to 30 kGy.

7. The method of any one of claims 1 to 6, wherein the sterilizing step includes sterilizing a biosensor-based medical device with a biosensor based reagent composition that has an analyte specific enzyme and a mediator.

8. The method of claim 7, wherein the analyte specific enzyme includes PQQ (pyrroloquinoline quinone) and the mediator includes ferricyanide.

**Patentansprüche**

1. Verfahren zur Herstellung von sterilisierten und kalibrierten integrierten biosensoren-basierten medizinischen Einwegvorrichtungen, wobei das Verfahren Folgendes umfasst:

   Zusammenbauen einer Vielzahl von integrierten biosensoren-basierten medizinischen Einwegvorrichtungen, die jeweils einen Biosensor und eine integrierte Lanzette aufweisen, wobei der Biosensor eine Biosensor-Reagenzzusammensetzung aufweist;
   Verpacken der integrierten biosensoren-basierten medizinischen Einwegvorrichtungen, wodurch verpackte integrierte biosensoren-basierte medizinische Einwegvorrichtungen erzeugt werden;
   anschließendes Sterilisieren der zusammengebauten integrierten biosensoren-basierten medizinischen Einwegvorrichtungen, wodurch eine Vielzahl von sterilisierten, verpackten integrierten biosensoren-basierten medizinischen Einwegvorrichtungen erzeugt wird;
   anschließendes Kompensieren der Wirkungen des Sterilisationsschrittes auf die analytische Leistung der verpackten integrierten biosensoren-basierten medizinischen Einwegvorrichtungen durch Kalibrieren der Biosensor-Reagenzzusammensetzung der sterilisierten, verpackten integrierten biosensoren-basierten medizinischen Einwegvorrichtungen, wobei der Kalibrationsschritt einen Teil der sterilisierten, verpackten integrierten biosensoren-basierten medizinischen Einwegvorrichtungen verwendet, um Kalibrationsinformationen zu erhalten.

2. Verfahren nach Anspruch 1, worin die integrierten medizinischen Vorrichtungen mit Biosensor und Lanzette elektrochemische biosensoren-basierte medizinische Vorrichtungen sind.

3. Verfahren nach Anspruch 1, worin die integrierten medizinischen Vorrichtungen mit Biosensor und Lanzette photometrische biosensoren-basierte medizinische Vorrichtungen sind.

4. Verfahren nach einem der Ansprüche 1 bis 3, worin der Sterilisationsschritt eine auf Strahlung basierende Sterilisationstechnik verwendet.

5. Verfahren nach Anspruch 4, worin der Sterilisationsschritt eine auf Gammastrahlung basierende Sterilisationstechnik verwendet.

6. Verfahren nach Anspruch 4, worin der Sterilisationsschritt eine Gammastrahlungsdosis im Bereich von 10 kGy bis 30 kGy verwendet.

7. Verfahren nach einem der Ansprüche 1 bis 6, worin der Sterilisationsschritt die Sterilisation einer biosensoren-basierten medizinischen Vorrichtung mit einer biosensoren-basierten Reagenzzusammensetzung umfasst, die ein analyten-spezifisches Enzym und einen Mediator aufweist.

8. Verfahren nach Anspruch 7, worin das analytenspezifische Enzym PQQ (Pyrrolochinolinchinon) umfasst und der Mediator Ferricyanid umfasst.

**Revendications**

1. Procédé pour fabriquer des dispositifs médicaux à biocapteur intégré jetables calibrés et stérilisés, le procédé comprenant les étapes suivantes :

   Assembler une pluralité de dispositifs médicaux à biocapteur intégré jetables, chacun comprenant un biocapteur et une lancette intégrés ensemble à celui-ci, le biocapteur comprenant une composition réactive de biocapteur ;
   empaqueter les dispositifs médicaux à biocapteur intégré jetables, créant ainsi des dispositifs médicaux à biocapteur intégré jetables empaquetés ;
   puis stériliser les dispositifs médicaux à biocapteur intégré jetables empaquetés assemblés, créant ainsi une pluralité de dispositifs médicaux à biocapteur intégré jetables empaquetés, stérilisés ;
   puis, compenser les effets de l'étape de stérilisation sur la performance analytique des dispositifs médicaux à biocapteur intégré jetables empaquetés en calibrant la composition réactive de biocapteur des dispositifs médicaux à biocapteur intégré jetables empaquetés, stérilisés, l'étape de calibrage utilisant une fraction des dispositifs médicaux à biocapteur intégré jetables empaquetés, stérilisés pour obtenir des informations de calibrage.

**2.** Procédé selon la revendication 1, dans lequel les dispositifs médicaux à lancette et à biocapteur intégrés sont des dispositifs médicaux à biocapteur électrochimique.

**3.** Procédé selon la revendication 1, dans lequel les dispositifs médicaux à lancette et biocapteur intégrés sont des dispositifs médicaux à biocapteur photométrique.

**4.** Procédé selon l'une quelconque des revendications 1 à 3, dans lequel l'étape de stérilisation utilise une technique de stérilisation à base de rayonnements.

**5.** Procédé selon la revendication 4, dans lequel l'étape de stérilisation utilise une technique de stérilisation à base de rayonnements gamma.

**6.** Procédé selon la revendication 4, dans lequel l'étape de stérilisation utilise une dose de rayonnements gamma située dans une plage allant de 10 kGy à 30 kGy.

**7.** Procédé selon l'une quelconque des revendications 1 à 6, dans lequel l'étape de stérilisation comprend de stériliser un dispositif médical à biocapteur avec une composition réactive de biocapteur qui a une enzyme spécifique à un analyte et un médiateur.

**8.** Procédé selon la revendication 7, dans lequel l'enzyme spécifique à un analyte comprend du PQQ (pyrroloquinoline quinone) et le médiateur comprend du ferricyanure.

100

102

104

106

108

**FIG. 1**
**(Prior Art)**

200

206

202

204

208

**FIG. 2**
**(Prior Art)**

/-300

/-310

STERILIZING AT LEAST ONE BIOSENSOR-
BASED MEDICAL DEVICE THAT INCLUDES
A BIOSENSOR REAGENT COMPOSITION

/-320

CALIBRATING THE BIOSENSOR REAGENT
COMPOSITION OF THE AT LEAST ONE
BIOSENSOR-BASED MEDICAL DEVICE

**FIG. 3**

/-400

/-410

ASSEMBLE A PLURALITY OF BIOSENSOR-
BASED MEDICAL DEVICES THAT INCLUDE
A BIOSENSOR REAGENT COMPOSITION

/-420

PACKAGE THE BIOSENSOR-BASED
MEDICAL DEVICES

/-430

STERILIZE THE PACKAGED BIOSENSOR-
BASED MEDICAL DEVICES USING A
RADIATION BASED TECHNIQUE

/-440

CALIBRATE THE BIOSENSOR REAGENT
COMPOSITION OF THE STERILIZED
PACKAGED BIOSENSOR-BASED MEDICAL
DEVICES

**FIG. 4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20020168290 A1
- EP 1360931 A
- EP 03255680 A

### Non-patent literature cited in the description

- **M. COLLISON et al.** *Clinical Chemistry,* 1999, vol. 45 (9), 1665-1673